# EUROPEAN PATENT APPLICATION

(11) **EP 4 659 652 A1**
(43) Date of publication of application: **10.12.2025**
(21) Application number: 24763679.8
(22) Date of filing: 19.02.2024
(51) Int. Cl.: A61B 3/00

(54) **FOREHEAD BAND, AND OPHTHALMOLOGIC DEVICE**

(30) Priority: 02.03.2023 JP 2023031840
(71) Applicant: Topcon Corporation, Tokyo 174-8580 (JP)
(72) Inventor: SHIMADA Sora, Tokyo 174-8580 (JP); TSURUMAKI Takeshi, Tokyo 174-8580 (JP); YOSHIDA Yuji, Tokyo 174-8580 (JP)
(74) Representative: Schmitt-Nilson Schraud Waibel Wohlfrom Patentanwälte Partnerschaft mbB
(86) International application number: PCT/JP2024/005706
(87) International publication number: WO 2024/181198

(57) **Abstract**

There are provided a forehead rest which allows a subject to easily align his/her forehead with a proper position with respect to the forehead rest and an ophthalmic device including the forehead rest. A forehead rest (14c) which is provided in an ophthalmic device (a slit lamp microscope (10)) and against which a forehead of a subject (H) is to be put includes: a forehead-contacting face (64) which is to contact the forehead and left and right edge-forming faces (66) which connect to two end sides in a left-right direction (X direction) of the forehead-contacting face (64) to form left and right edge portions (70) on the two end sides of the forehead-contacting face (64) between the edge-forming faces and the forehead-contacting face (64).

## Description

### {Technical Field}

The present disclosure relates to a forehead rest which is to support a forehead of a subject and an ophthalmic device including the forehead rest.

### {Background Art}

A slit lamp microscope (also called a slit lamp) is known as an ophthalmic device to be installed in an ophthalmic clinic. Such a slit lamp microscope uses slit light to pick up an optical slice of a region of interest of a subject eye, thereby achieving observation of a cross-section of the region of interest and acquiring an image of the cross-section (see Patent Literatures 1 and 2). According to this, an examiner can observe the region of interest of the subject eye.

The slit lamp microscopes described in Patent Literatures 1 and 2 are each provided with a face support for fixing a face of a subject during observation of a region of interest of a subject eye by an examiner. The face support generally includes a chin rest which is to support a chin of a subject and a forehead rest against which a forehead of the subject is to be put.

### {Citation List}

### {Patent Literature}

Patent Literature 1: Japanese Patent Application Laid-Open No. 2008-259544
Patent Literature 2: Japanese Patent Application Laid-Open No. 2014-23868

### {Summary of Invention}

### {Technical Problem}

In a case where a subject puts his/her forehead against a forehead rest described in each of Patent Literatures 1 and 2, a position of the forehead of the subject may be shifted in a left-right direction from a proper position with respect to the forehead rest. In this state with the position of the forehead shifted in the left-right direction, an optical axis of a slit lamp microscope may fail to be aligned with a subject eye or a face of the subject may move during observation of a region of interest of the subject eye by an examiner.

The present disclosure has been made in view of the above-described circumstances, and has as its object to provide a forehead rest which allows a subject to easily align his/her forehead with a proper position with respect to the forehead rest and an ophthalmic device including the forehead rest.

### {Solution to Problem}

A forehead rest for achieving the object of the present disclosure is a forehead rest which is provided in an ophthalmic device and against which a forehead of a subject is to be put, including a forehead-contacting face configured to contact the forehead and left and right edge-forming faces which connect to two end sides in a left-right direction of the forehead-contacting face to form left and right edge portions on the two end sides of the forehead-contacting face between the left and right edge-forming faces and the forehead-contacting face, respectively.

According to the forehead rest, one of the left and right edge portions can contact the forehead with an edge angled with respect to the forehead in a case where the forehead of the subject is shifted in the left-right direction from a proper position with respect to the forehead-contacting face.

In the forehead rest according to another aspect of the present disclosure, the left and right edge portions are in contact with the forehead at the forehead-contacting face forming the edge portions in a proper state where the forehead is at a proper position with respect to the forehead-contacting face, and one of the left and right edge portions is in contact with the forehead with an edge more highly angled with respect to the forehead than in the proper state in a misaligned state where the forehead is shifted in the left-right direction from the proper state with respect to the forehead-contacting face. Thereby, the subject can recognize that a position of the forehead is shifted in the left-right direction from the proper position.

The forehead rest according to another aspect of the present disclosure includes a forehead rest body at which the forehead-contacting face and the left and right edge-forming faces are formed.

In the forehead rest according to another aspect of the present disclosure, the forehead rest body includes a body front surface and a protruding portion which protrudes from a part of the body front surface toward a front side of the body front surface and has an upper surface and side surfaces, and the forehead-contacting face is the upper surface, and the left and right edge-forming faces are left and right ones of the side surfaces of the protruding portion.

In the forehead rest according to another aspect of the present disclosure, the forehead rest body includes a body front surface and a depressed face which is formed in a depressed manner at a part of the body front surface, the forehead-contacting face includes the depressed face, and the left and right edge-forming faces include regions on the two end sides of the forehead-contacting face in the body front surface.

In the forehead rest according to another aspect of the present disclosure, the forehead-contacting face includes a concave surface.

In the forehead rest according to another aspect of the present disclosure, the forehead-contacting face is tilted toward a front side of the forehead-contacting face in side view as viewed in the left-right direction.

In the forehead rest according to another aspect of the present disclosure, the forehead-contacting face is a mirror-finished surface. This configuration increases the degree of close contact (a feeling of close contact) when the subject brings the forehead into contact with the forehead-contacting face and makes it easier for the subject to recognize separation of the forehead from the forehead-contacting face. As a result, the subject more easily recognizes a lateral shift of the forehead with respect to the forehead-contacting face. A texture of the forehead-contacting face when the subject brings the forehead into contact with the forehead-contacting face can be improved.

An ophthalmic device for achieving the object of the present disclosure includes the above-described forehead rest.

### {Advantageous Effects of Invention}

The present disclosure allows a subject to easily align his/her forehead with a proper position with respect to a forehead rest.

### {Brief Description of Drawings}

Figure 1 is a side view of a slit lamp microscope;
Figure 2 is an external perspective view of a face support as viewed from a subject side;
Figure 3 is an external perspective view of a forehead rest as viewed from the subject side;
Figure 4 is a cross-sectional view of the forehead rest taken along a YZ plane at a center position in an X direction;
Figure 5 is an explanatory view for explaining states of contact of left and right edge portions with a forehead of a subject in a case where the forehead is in contact with a forehead-contacting face at a proper position;
Figure 6 is an explanatory view for explaining the states of contact of the left and right edge portions with the forehead in a case where the forehead of the subject is shifted in the X direction from the proper position with respect to the forehead-contacting face;
Figure 7 is an explanatory view for explaining optimum shapes of the left and right edge portions;
Figure 8 is a top view of a forehead rest of a slit lamp microscope according to a second embodiment;
Figure 9 is an explanatory view for explaining states of contact of left and right edge portions with a forehead of a subject in a proper state; and
Figure 10 is an explanatory view for explaining the states of contact of the left and right edge portions with the forehead of the subject in a misaligned state.

### {Description of Embodiments}

### [First Embodiment]

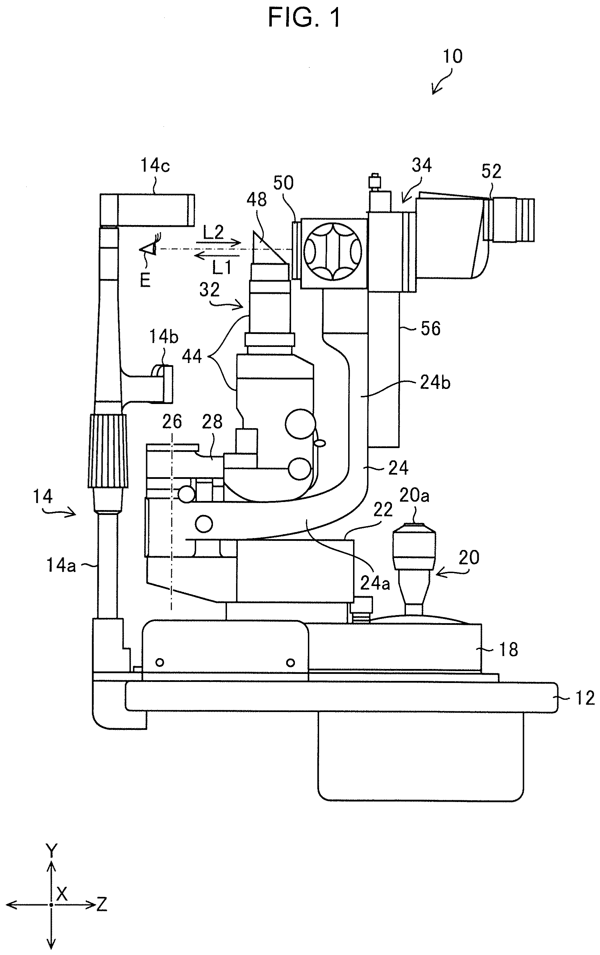

Figure 1 is a side view of a slit lamp microscope 10. The slit lamp microscope 10 corresponds to an ophthalmic device according to the present disclosure and is used for various types of observations, such as cornea observation, fundus observation, and crystalline lens observation, of a subject eye E. The slit lamp microscope 10 is also used for corneal endothelial cell observation that directly observes corneal endothelial cells of a cornea of the subject eye E. Note that an X direction in Figure 1 is a left-right direction (an eye width direction of the subject eye E) based on a subject (patient) and that a Y direction is an up-down direction. A Z direction orthogonal to both the X direction and the Y direction is a front-back direction (operating distance direction) parallel to a forward direction toward the subject and a rearward direction away from the subject.

As illustrated in Figure 1, the slit lamp microscope 10 is of the publicly known Zeiss type (Littman type). The slit lamp microscope 10 includes a base 12, a face support 14, a movable table 18, a manipulation lever 20, a support member 22, a microscope supporting arm 24, a pivoting axis 26, an illumination support arm 28, an illumination system 32, and a microscope 34.

The base 12 is placed on an eye examination table (not illustrated). The face support 14 is provided on an upper surface of the base 12 and at a front end portion on a subject side (subject eye E side). On the upper surface of the base 12, the movable table 18 is held so as to be movable in the X and Z directions (the left-right direction and the front-back direction).

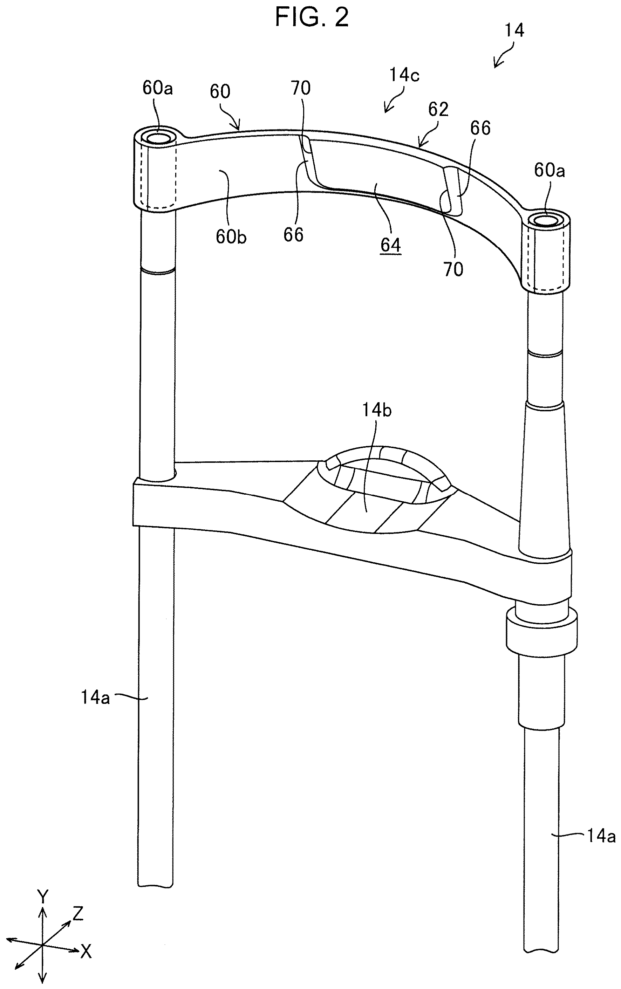

Figure 2 is an external perspective view of the face support 14 as viewed from the subject side. As illustrated in Figure 2, the face support 14 has one pair of columns 14a which are fixed to the base 12 and extend in the Y direction, a chin rest 14b which is provided at an intermediate portion between the pair of columns 14a, and a forehead rest 14c which is provided at upper end portions of the pair of columns 14a. When the subject puts his/her chin against the chin rest 14b and his/her forehead against the forehead rest 14c, a face of the subject is supported by the face support 14. With this support, a position of the subject eye E is fixed.

Referring back to Figure 1, the manipulation lever 20 is provided on an upper surface of the movable table 18 and at a rear end portion on a rearward-direction side (examiner side). On the upper surface of the movable table 18, the support member 22 is further provided so as to be movable in the Y direction (movable up and down).

The manipulation lever 20 is a manipulation member for manual movement manipulation of the support member 22 (the illumination system 32 and the microscope 34) in each of the X, Y, and Z directions. For example, the movable table 18 is manually moved in the Z direction or the X direction by movement manipulation or tilting manipulation of the manipulation lever 20 in the Z direction or the X direction. By pivoting manipulation of the manipulation lever 20 around an axis thereof, the support member 22 is moved in the Y direction via a drive transmission mechanism (not illustrated). Note that a switch 20a used for, e.g., photographing is provided at a top of the manipulation lever 20.

The pivoting axis 26 extending in the Y direction is provided at an end portion on a forward-direction side of the support member 22, and the microscope supporting arm 24 and the illumination support arm 28 are attached to the pivoting axis 26. The microscope supporting arm 24 is formed in a generally L-shape and has a horizontal arm portion 24a and a vertical arm portion 24b.

An end portion on the forward-direction side of the horizontal arm portion 24a is attached onto the support member 22 via the pivoting axis 26 extending in the Y direction so as to be horizontally pivotable. The illumination support arm 28 is attached onto the horizontal arm portion 24a via the pivoting axis 26 so as to be horizontally pivotable. As can be seen from this, the microscope supporting arm 24 and the illumination support arm 28 are pivotally attached to the common pivoting axis 26.

Note that horizontal pivoting of the microscope supporting arm 24 and the illumination support arm 28 around the pivoting axis 26 may be manually performed by the examiner or may be electrically performed using an electric pivoting mechanism (not illustrated).

The microscope 34 is attached to an upper end portion of the vertical arm portion 24b. The illumination support arm 28 is provided with the illumination system 32.

The illumination system 32 includes a slit lamp 44 and a deflection optical element 48. The slit lamp 44 is attached to the illumination support arm 28 and emits illumination light L1 toward the deflection optical element 48. A light source and an optical system (both not illustrated) for emitting the illumination light L1 toward the deflection optical element 48 are housed in the slit lamp 44.

The deflection optical element 48 is a prism or a mirror which is provided at a position above the slit lamp 44. The deflection optical element 48 performs deflection (including reflection) of the illumination light L1 emitted from the slit lamp 44 toward the subject eye E. With this deflection, the slit-like illumination light L1 (also referred to as slit light) is applied from the deflection optical element 48 to the subject eye E. The deflection optical element 48 is formed in a shape which prevents return light L2 from the subject eye E from being obscured. With this configuration, the return light L2 from the subject eye E passes by the deflection optical element 48 to enter the microscope 34.

Note that the slit lamp 44 is not limited to the one illustrated in Figure 1 and that a shape, a structure, and arrangement of the slit lamp 44 are not particularly limited as long as the slit lamp 44 is one used in the slit lamp microscope 10 of the Zeiss type.

The illumination system 32 is horizontally pivoted integrally with the illumination support arm 28 around the pivoting axis 26. Thereby, a direction in which the illumination light L1 is applied to the subject eye E can be adjusted.

The microscope 34 is used for, e.g., observation of the subject eye E. When the illumination light L1 is applied from the illumination system 32 to the subject eye E, the return light L2 from the subject eye E enters the microscope 34. An objective lens 50 is provided at a front end portion on the forward-direction side (the subject eye E side in the Z direction) of the microscope 34, and an eyepiece 52 is provided at a rear end portion on the rearward-direction side (the examiner side in the Z direction). A publicly known optical system (not illustrated) for observation of the subject eye E is housed inside the microscope 34.

The microscope 34 is horizontally pivoted integrally with the microscope supporting arm 24 around the pivoting axis 26. Thereby, a direction in which the subject eye E is observed by the microscope 34 can be adjusted. The microscope 34 is also provided with a digital camera 56 which photographs the subject eye E via the optical system of the microscope 34.

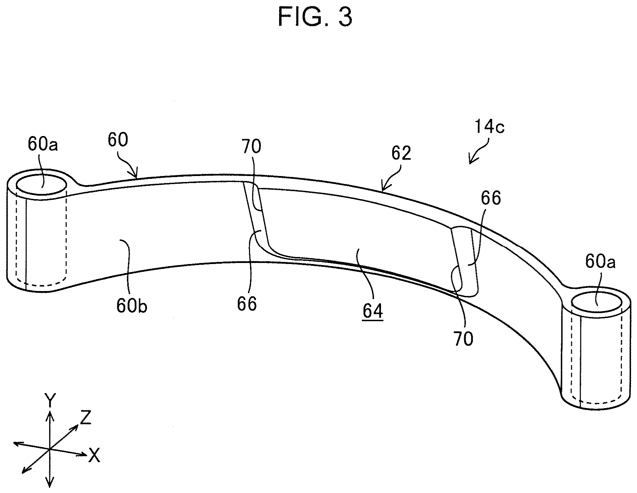

Figure 3 is an external perspective view of the forehead rest 14c as viewed from the subject side. As illustrated in Figure 3 and Figure 2, the forehead rest 14c includes a forehead rest body 60, a protruding portion 62, a forehead-contacting face 64, and left and right edge-forming faces 66.

The forehead rest body 60 is formed in a generally curved plate shape convex toward a rear side in the Z direction which is a side with a body (ophthalmic device body) of the slit lamp microscope 10, including the illumination system 32 and the microscope 34, when viewed from the Y direction. Note that a shape of the forehead rest body 60 is not particularly limited and that the forehead rest body 60 may be formed in, for example, a flat plate shape.

Mounting holes 60a, to which the upper end portions of the pair of columns 14a are to be attached, are formed at two end portions in the X direction (left-right direction) of the forehead rest body 60. The forehead rest body 60 also has a body front surface 60b which is to face the forehead of the subject. The body front surface 60b has a generally concave surface shape which follows a front surface side of a head of the subject. At a middle portion in the X direction which is a part of the body front surface 60b, the protruding portion 62 protruding toward the subject side (a front side in the Z direction) that is a front side of the middle portion is formed together with the forehead rest body 60.

The protruding portion 62 is formed in a generally curved plate shape which is thick on the front side in the Z direction and follows a shape of the body front surface 60b at the middle portion in the X direction of the body front surface 60b. An upper surface on the front side in the Z direction of the protruding portion 62 is the forehead-contacting face 64 that is to contact the forehead of the subject. Side surfaces on two end sides (on the left and right) in the X direction of the protruding portion 62 are the left and right edge-forming faces 66 that connect to two end sides in the X direction of the forehead-contacting face 64.

The forehead-contacting face 64 is formed at a position on the front side in the Z direction of the body front surface 60b. The forehead-contacting face 64 is formed in a shape which fits with a curved surface shape of the forehead of the subject and a shape capable of supporting the face of the subject, i.e., to have a concave surface. Since the forehead-contacting face 64 is to contact the forehead, the forehead-contacting face 64 is made of a biocompatible material.

The forehead-contacting face 64 is further formed to be a mirror-finished surface. This configuration increases the degree of close contact (a feeling of close contact) when the subject brings the forehead into contact with the forehead-contacting face 64 and makes it easier for the subject to recognize separation of the forehead from the forehead-contacting face 64. As a result, the subject more easily recognizes a lateral shift of the forehead with respect to the forehead-contacting face 64. A texture of the forehead-contacting face 64 when the forehead contacts the forehead-contacting face 64 can be improved. Note that a rubber film or a silicon film may be pasted or non-woven fabric may be removably pasted onto the forehead-contacting face 64.

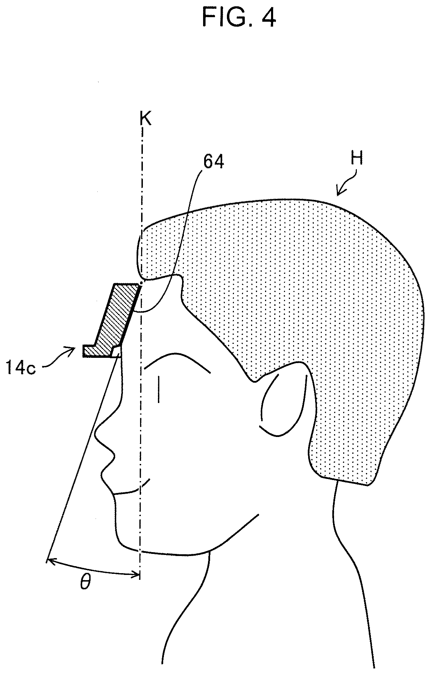

Figure 4 is a cross-sectional view of the forehead rest 14c taken along a YZ plane at a center position in the X direction of the forehead rest 14c. Note that reference character H in Figure 4 denotes the subject. As illustrated in Figure 4, the forehead-contacting face 64 has a generally straight line shape in side view as viewed from the X direction (left-to-right direction) and is tilted toward the front side in the Z direction so as to follow the forehead of the subject H. Letting K be a straight line passing through a center position in the X direction at an upper end of the forehead-contacting face 64 and parallel to the Y direction, a tilting angle θ of the forehead-contacting face 64 with respect to the straight line K is, for example, 18°.

Referring back to Figures 2 and 3, the left and right edge-forming faces 66 are tilted surfaces which connect the two end sides in the X direction of the forehead-contacting face 64 and the body front surface 60b and are tilted rearward in the Z direction with respect to the forehead-contacting face 64. Left and right edge portions 70 are formed by the forehead-contacting face 64 and the left and right edge-forming faces 66 on the two end sides in the X direction of the forehead-contacting face 64.

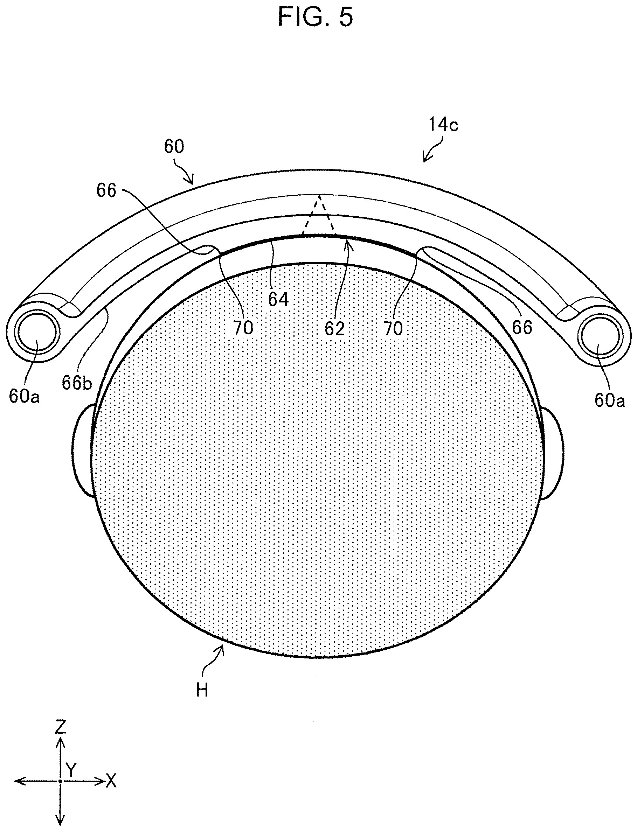

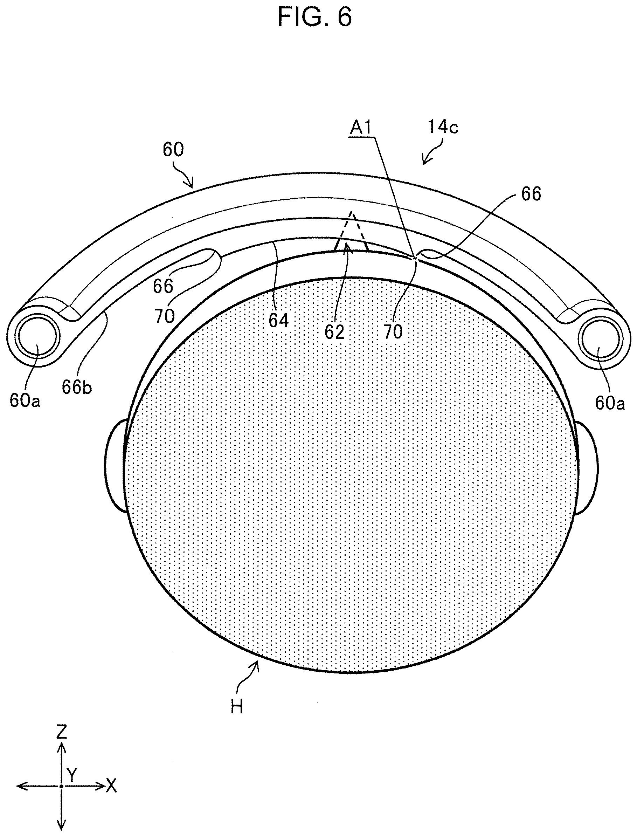

Figure 5 is an explanatory view for explaining states of contact of the left and right edge portions 70 with the forehead in a case where the forehead of the subject H is in contact with the forehead-contacting face 64 at a proper position. Figure 6 is an explanatory view for explaining the states of contact of the left and right edge portions 70 with the forehead in a case where the forehead of the subject H is shifted in the X direction from the proper position with respect to the forehead-contacting face 64.

As illustrated in Figure 5, the left and right edge portions 70 are each in surface contact with the forehead only at the forehead-contacting face 64 constituting the edge portion 70 in a proper state where the forehead of the subject H is in contact with the forehead-contacting face 64 at the proper position (hereinafter abbreviated as the proper state). That is, the left and right edge portions 70 contact the forehead with edges not angled with respect to the forehead. This makes it harder for the subject H to recognize contact of the forehead with the left and right edge portions 70 and makes it possible to give the subject H the sense that the forehead fits with the forehead-contacting face 64 at the proper position.

As illustrated in Figure 6, one (the edge portion 70 on a side toward which the shift direction heads) of the left and right edge portions 70 is in contact with the forehead with an edge more highly angled with respect to the forehead than in the proper state (see a leader line A1) in a misaligned state where the forehead of the subject H is shifted in the X direction (left-right direction) from the proper state illustrated in Figure 5 with respect to the forehead-contacting face 64 (hereinafter abbreviated as the misaligned state). Since the subject H recognizes contact with the edge portion 70, the subject H can recognize that a position of the forehead is shifted toward a one-direction side in the X direction as compared to the proper state.

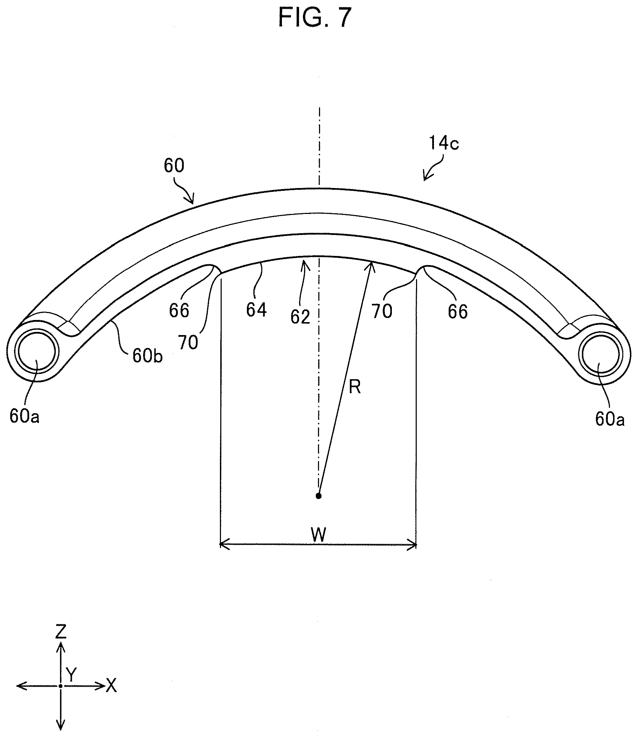

Figure 7 is an explanatory view for explaining optimum shapes of the left and right edge portions 70 (the forehead-contacting face 64). As illustrated in Figure 7, the present inventor found shapes of the left and right edge portions 70 which do not let the subject H recognize contact with the left and right edge portions 70 in the proper state and allow the subject H to recognize contact with one of the left and right edge portions 70 in the misaligned state, as a result of repeated execution of experiments and simulations with foreheads of various subjects H in mind. Specifically, the present inventor set a X-direction width W (a width in the left-right direction) between the left and right edge portions 70, i.e., the X-direction width W of the forehead-contacting face 64 at 70 mm. A curvature radius R of the forehead-contacting face 64 constituting the left and right edge portions 70 at 100 mm.

As described above, in the slit lamp microscope 10 according to the first embodiment, the left and right edge portions 70 that contact the forehead of the subject H with the edges not angled with respect to the forehead in the proper state and contact the forehead with an edge angled with respect to the forehead in the misaligned state are provided at two ends in the X direction of the forehead-contacting face 64. With this configuration, the subject H is capable of easily determining whether the position of the forehead with respect to the forehead-contacting face 64 is proper. For this reason, in a case where the misaligned state is created, it is possible to prompt the subject H to revise the position of the forehead to the proper position. As a result, the subject H can align the forehead with the proper position of the forehead-contacting face 64.

Since the left and right edge portions 70 only need to be provided at the two ends in the X direction of the forehead-contacting face 64 in the forehead rest 14c, low costs can be achieved.

### [Second Embodiment]

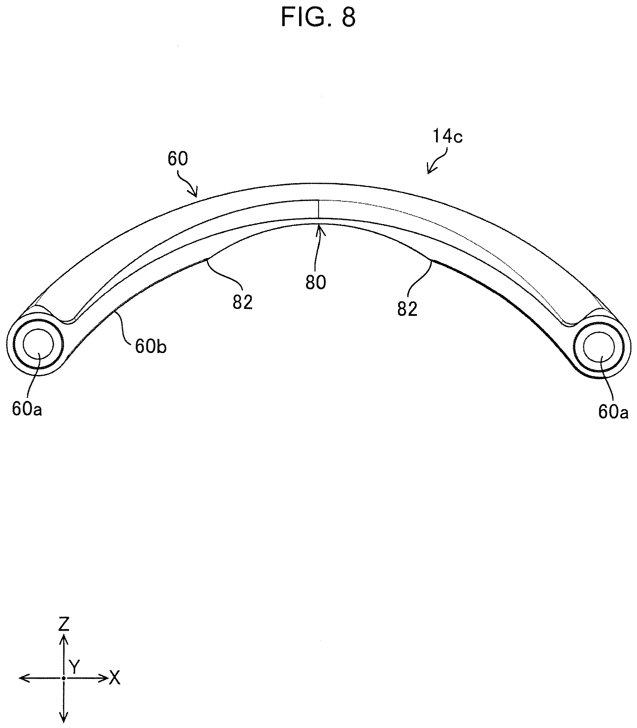

Figure 8 is a top view of a forehead rest 14c of a slit lamp microscope 10 according to a second embodiment. In the forehead rest 14c according to the above-described first embodiment, the forehead-contacting face 64, the left and right edge-forming faces 66, and the left and right edge portions 70 are formed by providing the protruding portion 62 at the body front surface 60b of the forehead rest body 60. In contrast, in the forehead rest 14c according to the second embodiment, left and right edge portions 82 are formed by depressing a body front surface 60b to form a forehead-contacting face 80.

Note that the slit lamp microscope 10 according to the second embodiment is basically the same in configuration as the slit lamp microscope 10 according to the first embodiment except that the forehead-contacting face 80 and the left and right edge portions 82 are formed at the forehead rest 14c. For this reason, components functionally or structurally identical to those in the first embodiment are denoted by identical reference numerals, and a description thereof will be omitted.

The forehead-contacting face 80 is a depressed face which is formed by depressing a middle portion in an X direction of the body front surface 60b rearward in a Z direction. The forehead-contacting face 80 is formed in a shape similar (in a concave surface, a mirror-finished shape, an X-direction width W, and a curvature radius R) to the forehead-contacting face 64 according to the first embodiment. With this configuration, the forehead-contacting face 80 can support a forehead of a subject H.

In the body front surface 60b according to the second embodiment, regions located on two end sides in the X direction of the forehead-contacting face 80 connect to two end portions in the X direction of the forehead-contacting face 80 and are tilted rearward in the Z direction relative to the two end portions in the X direction of the forehead-contacting face 80. For this reason, the left and right edge portions 82 are formed by the forehead-contacting face 80 and the body front surface 60b on the two end sides in the X direction of the forehead-contacting face 80.

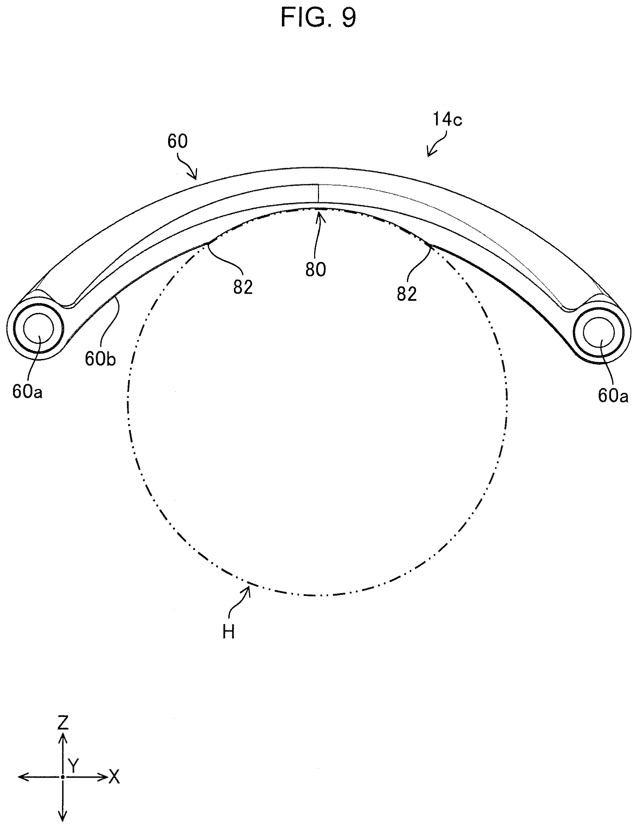

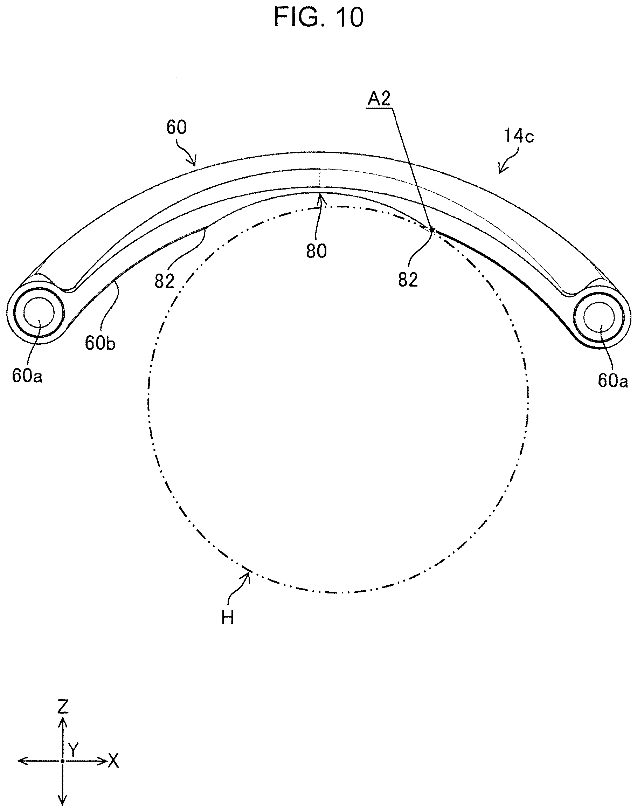

Figure 9 is an explanatory view for explaining states of contact of the left and right edge portions 82 with the forehead of the subject H in a proper state. Figure 10 is an explanatory view for explaining the states of contact of the left and right edge portions 82 with the forehead of the subject H in a misaligned state.

As illustrated in Figure 9, the left and right edge portions 82 are each in surface contact with the forehead only at the forehead-contacting face 80 constituting the edge portion 82 in the proper state. That is, the left and right edge portions 82 contact the forehead with edges not angled with respect to the forehead. This makes it possible to give the subject H the sense that the forehead fits with the forehead-contacting face 80 at a proper position, as in the first embodiment.

In contrast, as illustrated in Figure 10, one (the edge portion 82 on a side toward which a shift direction heads) of the left and right edge portions 82 is in contact with the forehead of the subject H with an edge more highly angled with respect to the forehead than in the proper state (see a leader line A2) in the misaligned state. Thereby, the subject H can recognize that a position of the forehead is shifted toward a one-direction side in the X direction as compared to the proper state, as in the first embodiment.

As described above, in the slit lamp microscope 10 according to the second embodiment as well, the left and right edge portions 82 contact the forehead of the subject H with the edges not angled with respect to the forehead in the proper state and contact the forehead with the edges angled with respect to the forehead in the misaligned state. With this configuration, the subject H is capable of easily determining whether the position of the forehead with respect to the forehead-contacting face 80 is proper. As a result, the same effects as those described in the first embodiment can be achieved.

### [Others]

In the above-described embodiments, the forehead rest 14c is provided so as to lie astride the upper end portions of the pair of columns 14a. However, for example, a rod-like support member may be connected on a back side of the forehead rest 14c (see, for example, Japanese Patent Application Laid-Open No. 2022-64300).

Although the slit lamp microscope 10 of the Zeiss type (Littman type), in which the slit lamp 44 (light source) of the illumination system 32 is provided below the deflection optical element 48 has been described as an example in each of the embodiments, the present disclosure can also be applied to the slit lamp microscope 10 of the Haag type (Goldmann type), in which the slit lamp 44 is provided above the deflection optical element 48).

Although the forehead rest 14c of the slit lamp microscope 10 has been described as an example in each embodiment, the present disclosure can also be applied to the forehead rest 14c that is provided in various types of ophthalmic devices (including surgical devices) used for observation of the subject eye E, acquisition of various ocular characteristics, surgery, and the like.

Various inventive concepts may be embodied as one or more processes, of which examples have been provided. The acts performed as part of each process may be ordered in any suitable way. Thus, embodiments may be constructed in which acts are performed in an order different than illustrated, which may include performing some acts simultaneously, even though shown as sequential acts in illustrative embodiments.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, for example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

Use of ordinal terms such as "first," "second," "third," etc., in the claims to modify a claim element does not by itself connote any priority, precedence, or order of one claim element over another or the temporal order in which acts of a method are performed. Such terms are used merely as labels to distinguish one claim element having a certain name from another element having a same name (but for use of the ordinal term). The phraseology and terminology used herein is for the purpose of description and should not be regarded as limiting. The use of "including," "comprising," "having," "containing," "involving," and variations thereof, is meant to encompass the items listed thereafter and additional items.

Having described several embodiments of the techniques described herein in detail, various modifications, and improvements will readily occur to those skilled in the art. Such modifications and improvements are intended to be within the spirit and scope of the disclosure. Accordingly, the foregoing description is by way of example only, and is not intended as limiting. The techniques are limited only as defined by the following claims and the equivalents thereto.

### {Reference Signs List}

10: slit lamp microscope
12: base
14: face support
14a: column
14b: chin rest
14c: forehead rest
16: electric drive unit
18: movable table
20: manipulation lever
20a: switch
22: support member
24: microscope supporting arm
24a: horizontal arm portion
24b: vertical arm portion
26: pivoting axis
28: illumination support arm 28
32: illumination system
34: microscope
38: handy manipulation unit
44: slit lamp
48: deflection optical element
50: objective lens
52: eyepiece
56: digital camera
60: forehead rest body
60a: mounting hole
60b: body front surface
62: protruding portion
64: forehead-contacting face
64a: normal
66: edge-forming face
70: edge portion
80: forehead-contacting face
82: edge portion
A1: leader line
A2: leader line
E: subject eye
H: subject
K: straight line
L1: illumination light
L2: return light
R: curvature radius
W: X-direction width
θ: tilting angle

## Claims

1. A forehead rest which is provided in an ophthalmic device and against which a forehead of a subject is to be put, comprising:
a forehead-contacting face configured to contact the forehead; and
left and right edge-forming faces which connect to two end sides in a left-right direction of the forehead-contacting face to form left and right edge portions on the two end sides of the forehead-contacting face between the left and right edge-forming faces and the forehead-contacting face, respectively.

2. The forehead rest according to claim 1, wherein
the left and right edge portions are in contact with the forehead at the forehead-contacting face forming the edge portions in a proper state where the forehead is at a proper position with respect to the forehead-contacting face, and
one of the left and right edge portions is in contact with the forehead with an edge more highly angled with respect to the forehead than in the proper state in a misaligned state where the forehead is shifted in the left-right direction from the proper state with respect to the forehead-contacting face.

3. The forehead rest according to claim 1, comprising
a forehead rest body at which the forehead-contacting face and the left and right edge-forming faces are formed.

4. The forehead rest according to claim 3, wherein
the forehead rest body includes
a body front surface, and
a protruding portion which protrudes from a part of the body front surface toward a front side of the body front surface and has an upper surface and side surfaces, and
the forehead-contacting face is the upper surface, and the left and right edge-forming faces are left and right ones of the side surfaces of the protruding portion.

5. The forehead rest according to claim 3, wherein
the forehead rest body includes a body front surface and a depressed face which is formed in a depressed manner at a part of the body front surface,
the forehead-contacting face includes the depressed face, and
the left and right edge-forming faces include regions on the two end sides of the forehead-contacting face in the body front surface.

6. The forehead rest according to any one of claims 1 to 5, wherein the forehead-contacting face includes a concave surface.

7. The forehead rest according to claim 6, wherein the forehead-contacting face is tilted toward a front side of the forehead-contacting face in side view as viewed in the left-right direction.

8. The forehead rest according to any one of claims 1 to 5, wherein the forehead-contacting face includes a mirror-finished surface.

9. An ophthalmic device comprising a forehead rest according to any one of claims 1 to 5.
